(19) Europäisches Patentamt
European Patent Office
Office européen des brevets

(11) **EP 3 613 290 B1**

(12) **EUROPÄISCHE PATENTSCHRIFT**

(45) Veröffentlichungstag und Bekanntmachung des Hinweises auf die Patenterteilung:
**27.09.2023   Patentblatt 2023/39**

(21) Anmeldenummer: **19192083.4**

(22) Anmeldetag: **16.08.2019**

(51) Internationale Patentklassifikation (IPC):
**A21C 1/14** (2006.01)        **A21C 1/02** (2006.01)

(52) Gemeinsame Patentklassifikation (CPC):
**A21C 1/02; A21C 1/1455; A21C 1/146; B01F 29/81; B01F 29/85; G01N 11/14; G01N 33/10;** G01N 2011/0046

(54) **VERFAHREN ZUM BETREIBEN EINER TEIGKNETVORRICHTUNG SOWIE TEIGKNETVORRICHTUNG**

DOUGH KNEADING MACHINE AND METHOD FOR OPERATING A DOUGH KNEADING MACHINE

PROCÉDÉ DE FONCTIONNEMENT D'UN DISPOSITIF DE PÉTRISSAGE DE LA PÂTE AINSI QUE DISPOSITIF DE PÉTRISSAGE DE LA PÂTE

(84) Benannte Vertragsstaaten:
**AL AT BE BG CH CY CZ DK EE ES FI FR GB GR HR HU IE IS IT LI LT LU LV MC MK MT NL NO PL PT RO RS SE SI SK SM TR**

(30) Priorität: **23.08.2018   DE 102018214278**

(43) Veröffentlichungstag der Anmeldung:
**26.02.2020   Patentblatt 2020/09**

(73) Patentinhaber: **WP Kemper GmbH**
**33397 Rietberg (DE)**

(72) Erfinder:
• **Jander, Christopher**
  **33378 Rheda-Wiedenbrück (DE)**
• **Oestersötebier, Felix**
  **33332 Gütersloh (DE)**

(74) Vertreter: **Rau, Schneck & Hübner**
**Patentanwälte Rechtsanwälte PartGmbB**
**Königstraße 2**
**90402 Nürnberg (DE)**

(56) Entgegenhaltungen:
**EP-A1- 0 428 241        JP-A- 2008 145 132**
**US-A- 4 747 690        US-A- 5 472 273**
**US-A- 5 906 432**

EP 3 613 290 B1

**Beschreibung**

Die vorliegende Patentanmeldung nimmt die Priorität der deutschen Patentanmeldung DE 10 2018 214 278.5 in Anspruch.

Die Erfindung betrifft ein Verfahren zum Betreiben einer Teigknetvorrichtung. Ferner betrifft die Erfindung eine Teigknetvorrichtung.

Eine Teigknetvorrichtung ist beispielsweise aus der DE 10 2010 042 542 A1 bekannt.

Die JP 2008 145 132 A offenbart eine Beeinflussung eines Teig-Knetvorgangs über eine zeitaufgelöste Drehmomentmessung. Ein ermittelter Drehmomentwert wird einer Frequenzanalyse unterzogen. Die US 4,747,690 offenbart eine Vorrichtung und ein Verfahren zur Überprüfung einer Brot-Teigqualität. Die US 5,472,273 offenbart ein System, welches den Entwicklungszustand von Teig innerhalb einer Knetvorrichtung bestimmt. Die US 5,906,432 offenbart einen Teigkneter für einen Laborbetrieb. Die EP 0 428 241 A1 offenbart eine Vorrichtung und ein Verfahren zum Teigkneten.

Es ist eine erste Aufgabe der vorliegenden Erfindung, ein Verfahren zum Betreiben einer Teigknetvorrichtung derart weiterzubilden, dass objektive, keine unerwünschten Abhängigkeiten vom jeweiligen Messverfahren aufweisende Teig-Zustandsdaten während des Betriebs gewonnen werden können.

Diese Aufgabe ist erfindungsgemäß gelöst durch ein Verfahren mit den im Anspruch 1 angegebenen Merkmalen.

Der Prozess der industriellen Teigknetung gliedert sich in der Regel in zwei Phasen. Zuerst wird eine heterogene Masse bestehend aus Rohzutaten, wie Wasser, Mehl und Salz bei niedrigen Drehzahlen der Knetspirale zu einer homogenen Teigmasse vermischt. Bei der nachfolgenden Knetung werden chemische Teigstrukturen gebildet und der Teig, bei höherer Drehzahl, zu einer visko-elastischen Masse geknetet. Hierbei gilt es, möglichst optimierte Werte einerseits für die Elastizität bzw. Steifigkeit und andererseits für die Viskosität des Teiges zu erreichen. Die Viskosität nimmt zuerst zu, bis sie, nach Einhaltung eines Maximalniveaus, bei Überknetung wieder sinkt. Ein entsprechender Verlauf gilt für die Elastizität. Optimierungskriterien bei der Prozessbeurteilung ergeben sich einerseits aufgrund von Anforderungen an die Produktqualität und andererseits aufgrund von Anforderungen an die Effizienz des Bearbeitungsprozesses.

Erfindungsgemäß wurde erkannt, dass der Teig im Rahmen der Knetzeitraum-Bestimmung z. B. als ein, an der Bottichwand gelagertes, FederDämpfer-System abstrahiert werden kann, das dem Knetwerkzeug eine Reaktionskraft entgegenstellt. Hierbei modelliert die Federsteifigkeit $c_d$ die Elastizität und die Dämpfung $d_d$ die Viskosität des Teiges. Weitere Parameter können, neben der Federsteifigkeit und der Dämpfung, ein Radius des Knetwerkzeugs und ein minimaler Spalt $s_{min}$ zwischen Bottichwand und Werkzeug sein.

Erfindungsgemäß wurde zudem erkannt, dass in eine Auswertung eines Knetwerkzeug-Drehmoment-Messergebnisses nicht nur das gemessene Knetwerkzeug-Drehmoment selbst, sondern auch eine Drehzahl und eine Drehposition des Knetwerkzeugs eingehen sollen. Werden auch die Drehzahl- und Drehpositions-Daten berücksichtigt, ist eine entsprechend feinere Auswertung der Drehmoment-Messergebnisse möglich. Eine objektive Überwachung des gekneteten Teigs während des Knetbetriebs ist somit möglich.

Durch eine Schwingungsanalyse der zeitaufgelösten Messdaten sind somit die objektiven Teig-Messgrößen Viskosität und Elastizität einer Bestimmung zugänglich. Anhand des so objektiv bestimmten Teigzustandes lässt sich die Wirkung des Knetbetriebes reproduzierbar bestimmen und die Teigknetvorrichtung entsprechend zur Erzeugung eines optimal gekneteten Teiges steuern. Sowohl ein Unterkneten, bei dem unerwünscht eine Viskosität des Teiges bzw. eine Elastizität des Teiges zu niedrig ist, als auch ein Überkneten, bei dem unerwünscht eine Viskosität des Teiges bzw. eine Elastizität des Teiges ein Maximum überschritten haben, sind vermieden.

Die Schwingungsanalyse kann unter Zuhilfenahme eines erweiterten Kalman-Filters erfolgen.

Eine Steuerung des Knetbetriebes nach Anspruch 2 nutzt die Vorteile der Bestimmung der Teig-Ist-Parameter.

Bei einem Ist-Wert/Soll-Wert-Vergleich nach Anspruch 3 können die Ist-Werte einerseits der Elastizität und andererseits der Viskosität in vorgegebener Weise gemeinsam ihren Soll-Werten nachgeführt werden. Im einfachsten Fall ist die Merit-Funktion ein Produkt oder ein Verhältnis der Teigparameter Elastizität und Viskosität. Je Merit-Funktion können die Teigparameter auch gewichtet eingehen und es können insbesondere auch weitere Parameter wie beispielsweise die Teigtemperatur eingehen. Im Sinne einer Mehrzieloptimierung (mehrkriteriellen Optimierung) kann mit den zur Verfügung stehenden Stellgrößen ein hinsichtlich der Vorgaben optimaler Kompromiss zwischen den konkurrierenden Zielen eingestellt werden (Pareto-Optimum). Um darauf aufbauend die konkrete Gestalt der Pareto-Front, die sich abhängig von den veränderlichen Parametern bzw. Störeinflüssen (z.B. Umgebungsbedingungen) ergibt, zu berücksichtigen, kann ein gewünschtes Zielgrößenverhältnis mithilfe eines Pareto-Reglers eingeregelt werden. Ein derartiger Pareto-Regler ist beschrieben im Fachartikel: Kessler, Jan Henning; Trächtler, Ansgar: Control of Pareto Points for Self-Optimizing Systems with Limited Objective Values. In: The 19th IFAC World Congress, Cape Town, South Africa, 24. - 29. Aug. 2014.

Insbesondere kann als Ergebnis des Vergleichs der Ist-Werte mit den Soll-Werten, insbesondere anhand der Merit-Funktion, eine Nachführung eines Soll-Verlaufs einer zeitlichen Abhängigkeit der Drehzahl des Knetwerkzeuges erfolgen.

Es ist eine weitere Aufgabe der vorliegenden Erfindung, ein Verfahren zum Betreiben einer Teigknetvorrichtung derart weiterzubilden, dass ein möglichst vollständiges Auskneten des Teiges bei gleichzeitiger Vermeidung insbesondere eines unerwünschten Überknetens des Teiges erreicht wird.

Diese Aufgabe ist erfindungsgemäß gelöst durch ein Verfahren mit den im Anspruch 4 angegebenen Schritten.

Die zeitaufgelöste Auswertung der Momentan-Messdaten erlaubt einen entsprechend genauen Rückschluss auf einen zu erwartenden Knetzeitraum bis zum Erreichen eines maximalen Drehmoments, welches beim Betrieb der Teigknetvorrichtung auf das Knetwerkzeug wirkt. Es kann also präzise auf den Zeitpunkt rückgeschlossen werden, an dem das maximale Drehmoment erreicht wird und an dem der Teig entsprechend ausgeknetet in optimaler Qualität vorliegt. Ausgehend von dem dann bestimmten, zu erwartenden Zeitraum bis zum Erreichen des maximalen Drehmoments kann entschieden werden, wann genau der Knetbetrieb beendet wird.

Eine Beendigung des Knetbetriebes nach Anspruch 5 führt zum Vorliegen von optimal ausgeknetetem Teig direkt nach Beendigung des Knetbetriebes. Der Teig steht dann für eine weitere Verarbeitung direkt zur Verfügung.

Eine Beendigung des Knetbetriebes nach Anspruch 6 definiert vor Ablauf des bestimmten Zeitraumes bis zum Erreichen des maximalen Drehmoments bietet die Möglichkeit, nach dem Knetzeitraum den Teig einer weiteren Verarbeitung zuzuführen, in der der Teig noch mechanisch weiter belastet wird, sodass ein Auskneten des Teiges zum Erreichen der optimalen Teigkonsistenz noch nach dem Knetzeitraum in der Teigknetvorrichtung stattfindet. Diese Beendigung des Knetbetriebes definiert vor Ablauf des bestimmten Knetzeitraumes erhöht somit die Flexibilität der Nutzung der Teigknetvorrichtung.

Die Nutzung mehrerer paralleler Bestimmungsalgorithmen nach Anspruch 7 ermöglicht die Bestimmung des zu erwartenden Knetzeitraumes insbesondere bei Nutzung einer großen Parameter-Bandbreite, was die Zusammensetzung des Teiges und die Knet-Betriebsbedingungen angeht. Es kann dann zur Bestimmung des zu erwartenden Knetzeitraumes zum Beispiel zwischen den Ergebnissen der verschiedenen Bestimmungsalgorithmen gemittelt werden. Alternativ oder zusätzlich können extreme Ergebnisse vor der Bestimmung des zu erwartenden Knetzeitraumes aussortiert werden. Auch eine gewichtete Mittelung ist möglich.

Eine demokratische Bestimmung des zu erwartenden Knetzeitraumes nach Anspruch 8 führt zu einer hohen Robustheit der Knetzeitraum-Bestimmung. Wenn beispielsweise drei unabhängige Bestimmungsalgorithmen jeweilige Knetzeitraum-Resultate erbringen, wobei zwei dieser Resultate nur gering abweichen und ein drittes Resultat stärker abweicht, gehen in die Knetzeitraum-Bestimmung nur die beiden Resultate ein, die lediglich gering voneinander abweichen.

Bestimmungsalgorithmen nach den Ansprüchen 9 und 10 haben sich aufgrund ihrer Genauigkeit und/oder Robustheit bewährt.

Eine Temperaturmessung nach Anspruch 11 stellt eine entscheidende Größe für ein Ende der Mischphase des Teiges vor dem Kneten dar und erhöht weiterhin die Verlässlichkeit der Knetzeitraum-Bestimmung. In die Bestimmung des zu erwartenden Knetzeitraumes kann eine Teigfüllstandsmessung eingehen. Hierüber kann eine Verlässlichkeit einer Knetzeitraum-Bestimmung weiter erhöht werden. Es kann eine Teigtemperatur und/oder eine Raumtemperatur gemessen werden und in die Knetzeitraum-Bestimmung eingehen.

Es wurde zudem erkannt, dass abhängig vom verwendeten Bestimmungsalgorithmus bei der Modellierung einer zeitlichen Abhängigkeit des auf das Knetwerkzeug wirkenden Drehmoments die Teigparameter Steifigkeit und Viskosität als zusätzliche Ergebnisse resultieren. Diese Teigdaten können dann für eine weitere Teigverarbeitung genutzt werden.

Es ist eine weitere Aufgabe der vorliegenden Erfindung, eine Teigknetvorrichtung anzugeben, mit der insbesondere das vorstehend beschriebene Betriebsverfahren durchgeführt werden kann.

Diese Aufgabe ist erfindungsgemäß gelöst durch eine Teigknetvorrichtung mit den im Anspruch 12 angegebenen Merkmalen.

In der Analyseeinheit der Teigknetvorrichtung kann die Bestimmung der Teig-Ist-Parameter, also insbesondere des Teig-Elastizitätsparameters und des Teig-Viskositätsparameters und/oder die Bestimmung des zu erwartenden Knetzeitraumes bis zum Erreichen eines maximalen Knetwerkzeug-Drehmoments durchgeführt werden. Aufgrund der Signalverbindung der Analyseeinheit mit dem Knetwerkzeugantrieb kann die Analyseeinheit den Knetwerkzeugantrieb steuern und insbesondere abhängig vom bestimmten Teigzustand und/oder abhängig vom bestimmten Knetzeitraum den Knetbetrieb für eine aktuell geknetete Teigcharge beenden.

Die Vorteile einer Teigknetvorrichtung aus den Ansprüchen 13 und 14 entsprechen denen, die vorstehend zusammen mit den Ansprüchen 8 und 9 bereits erläutert wurden.

Ausführungsbeispiele der Erfindung werden nachfolgend anhand der Zeichnung näher erläutert. In dieser zeigen:

Figur 1    eine Ausführung einer Bearbeitungsvorrichtung zum Kneten von Teig in einer teilweise interne Details preisgebenden und teilweise geschnittenen Seitenansicht;

Figur 2    einen Ausschnitt eines Teigbehälters in Form eines Bottichs zusammen mit einem als Wendel bzw. als Spirale ausgeführten Knetwerkzeug mit einer benachbarten Wand des Bottichs der Teigknetvorrichtung;

Figur 3    im Vergleich zur Figur 2 wiederum stärker schematisch einen Schnitt gemäß Linie III-III in Fig. 2, wobei zusätzliche Abstands-, Winkel-, Kraft- und Drehmomentgrößen eingezeichnet sind;

Figur 4    in einem Diagramm schematisch einen Ausschnitt eines zeitlichen Verlaufs einer Messung eines momentanen Drehmoments, welches auf das Knetwerkzeug der Teigknetvorrichtung nach den Figuren 1 und 2 wirkt;

Figur 5    ebenfalls in einem Diagramm den zeitlichen Verlauf eines an die Messwerte nach Figur 4 angepassten, modellierten Drehmoments, welches auf das Knetwerkzeug wirkt;

Figur 6    schematisch ein Ablaufschema eines Verfahrens zum Betreiben der Teigknetvorrichtung nach den Figuren 1 bis 3; und

Figur 7    stark schematisch ein Diagramm, welches einen typischen, geglätteten Verlauf des auf das Knetwerkzeug wirkenden Drehmoments während eines Knetzeitraums bis zum Erreichen eines maximalen Drehmoments zeigt.

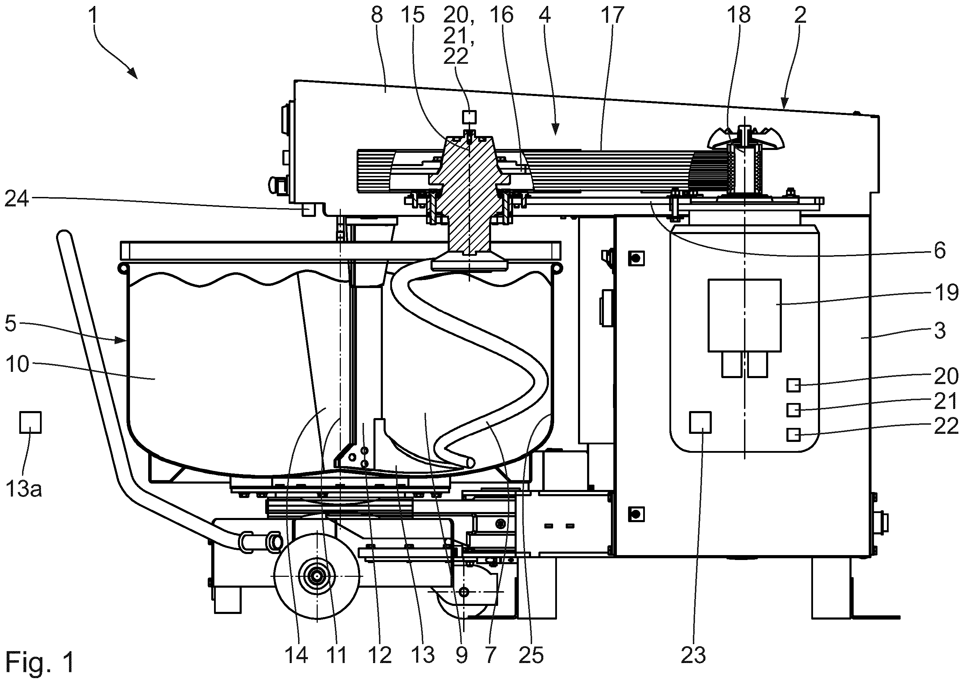

Anhand der Figuren 1 bis 3 wird nachfolgend eine Ausführung einer Bearbeitungsvorrichtung 1 zum Mischen und Kneten von Teig, also einer Teigknetvorrichtung, beschrieben. Die Bearbeitungsvorrichtung 1 hat einen Tragrahmen innerhalb eines Maschinengehäuses 2 mit einem Ständer 3 und einem Arm 4. Ein Bottich 5 der Bearbeitungsvorrichtung 1 dient zur Aufnahme des zu knetenden und zu mischenden Teiges. Der Bottich 5 stellt einen Teigbehälter dar.

An einer Rahmenplatte 6 des Tragrahmens, die innerhalb des Arms 4 verläuft, gelagert ist ein Knetwerkzeug 7 in Form einer vertikalen Knetwendel, die auch als Knetspirale bezeichnet ist. Das Knetwerkzeug 7 ragt in einer Arbeitsstellung, die in der Figur 1 dargestellt ist, ausgehend von einem über den Ständer 3 überstehenden Kopfabschnitt 8 des Arms 4 von oben her in den Bottich 5 hinein.

Ein Volumen innerhalb des sich drehenden Bottichs 5, das beim Betrieb des Knetwerkzeugs 7 von diesem geknetet und gemischt werden kann, gibt eine Knetzone 9 vor. Außerhalb der Knetzone 9 liegt der zu knetende und zu mischende Teig in einer Ruhezone 10 vor. Zwischen der Knetzone 9 und einer vertikalen Rotationssymmetrieachse 11 des Bottichs 5 ist ein Leitkörper 12 zum Leiten einer Bewegung des Teiges im Bottich 5 angeordnet. Am Leitkörper 12 ist ein Temperaturfühler bzw. Temperatursensor 13 des Typs Pt100 angebracht, der in direktem thermischen Kontakt mit dem Teig im Bottich 5 steht. Zudem hat die Teigknetvorrichtung 1 noch einen weiteren Temperatursensor 13a zur Bestimmung einer Raumtemperatur, bei der die Teigknetvorrichtung 1 betrieben wird.

Zwischen dem Leitkörper 12 und der Ruhezone 10 bildet sich im Betrieb der Bearbeitungsvorrichtung 1 eine vertikal verlaufende Luftsäule 14 innerhalb des Bottichs 5 aus.

Der Bottich 5 wird im Betrieb der Bearbeitungsvorrichtung 1 motorisch in an sich bekannter Weise um die Rotationssymmetrieachse 11 gedreht. Weiterhin wird das Knetwerkzeug 7 um eine zentrale, ebenfalls vertikal verlaufende Knetwendelachse 15, die beabstandet zur Bottich-Rotationssymmetrieachse 11 verläuft, motorisch angetrieben. Die Wendelachse 15 stellt gleichzeitig eine Knet-Drehachse dar, um die sich das Knetwerkzeug 7 beim Kneten dreht. Hierzu ist das Knetwerkzeug 7 drehfest mit einem Antriebsrad 16 verbunden. Um einen Teil des Umfangs des Antriebsrads 16 verläuft ein Antriebsriemen 17, über den das Antriebsrad 16 kraftschlüssig mit einer Antriebswelle 18 eines Knetantriebsmotors 19 verbunden ist. Der Knetantriebsmotor 19 stellt einen Knetwerkzeug-Antrieb der Teigkneteinrichtung dar. Auch der Knetantriebsmotor 19 ist an der Rahmenplatte 6 angebracht. Die Antriebswelle 18 verläuft parallel zur Wendelachse 15, also ebenfalls vertikal. Das Antriebsrad 16 und der Antriebsriemen 17 sind im Arm 4 des Maschinengehäuses 2 untergebracht. Der Knetantriebsmotor 19 ist im Ständer 3 des Maschinengehäuses 2 untergebracht.

Die Teigknetvorrichtung 1 hat weiterhin eine Drehmoment-Messeinheit 20 zum zeitaufgelösten Messen eines momentanen Drehmoments, der auf das Knetwerkzeug 7 wirkt. Die Drehmoment-Messeinheit 20 kann über die Messung einer Stromaufnahme des dann als Elektromotor ausgeführten Knetantriebsmotors 19 arbeiten. Alternativ kann das momentane Drehmoment, das auf das Knetwerkzeug 7 wirkt, auch mittels einer separaten Messeinheit erfasst werden.

Die Teigknetvorrichtung 1 hat weiterhin eine Drehzahl-Messeinheit 21 zum zeitaufgelösten Messen einer momentanen Drehzahl, mit der das Knetwerkzeug 7 rotiert, und eine Drehpositions-Messeinheit 22 zum zeitaufgelösten Messen einer momentanen Drehposition des Knetwerkzeugs 7.

Die Messeinheiten 20 bis 22 für das Drehmoment, die Drehzahl und die Drehposition können auch direkt im Bereich einer Lagerung des Knetwerkzeuges 7 angeordnet sein, wie in der Figur 1 schematisch angedeutet.

Die Drehpositions-Messeinheit 22 kann beispielsweise als Positionsgeber ausgeführt sein.

Eine Analyseeinheit 23 der Teigknetvorrichtung 1 steht mit der Drehmoment-Messeinheit 20 der Drehzahl-Messeinheit 21 und der Drehpositions-Messeinheit 22 in Signalverbindung. Die Analyseeinheit 23 ist zum Auswerten von Drehmoment-Messergebnissen der Drehmoment-Messeinheit 20 ausgeführt. Die Analyseeinheit 23 steht mit dem Knetantriebsmotor 19 in Signalverbindung.

Die Teigknetvorrichtung 1 hat weiterhin einen Füllstandssensor 24 zur Bestimmung eines Teig-Füllstandes im Teigbehälter 5. Bei dem Füllstandssensor 24 kann es sich um einen optischen Sensor handeln.

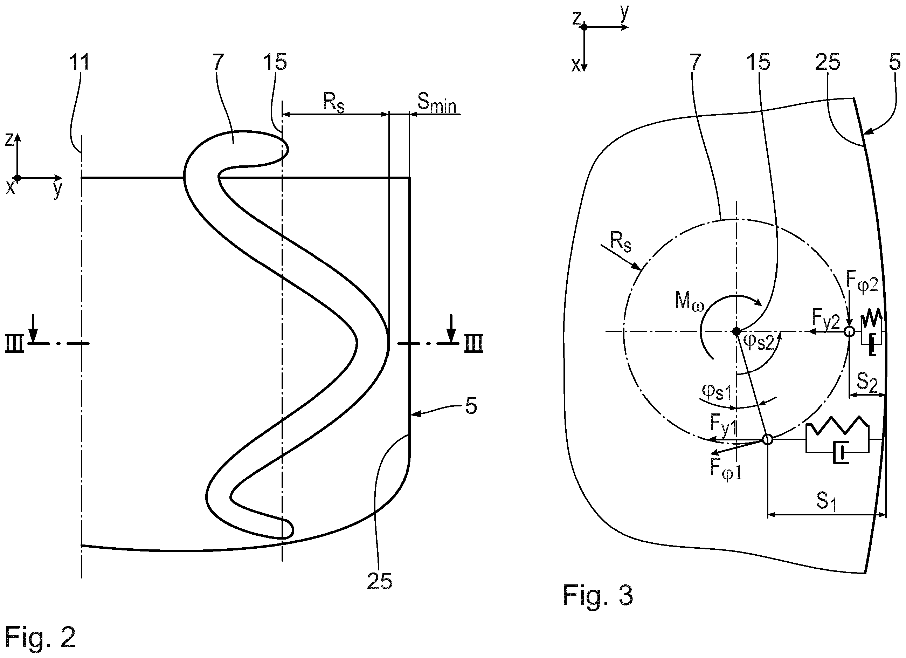

Der Knetvorgang, den das Knetwerkzeug 7 auf den zu knetenden Teig in Bottich 5 ausübt, kann über eine Modellierung des Teigs als Feder/Dämpferelement an einer Innenwand 25 des Bottichs 5 verstanden werden. Durch Aufstellung eines entsprechenden Systems von Differentialgleichungen kann eine Grundschwingung und eine erste Oberwelle eines zeitlichen Verlaufs des momentanen Drehmoments modelliert werden. Hierfür relevante Größen zeigen die Figuren 2 und 3.

Mit $R_s$ ist ein Radius des Knetwerkzeugs 7 im betrachteten Axialabschnitt, gemessen ausgehend von der Knetwinkelachse 15, bezeichnet. $S_{min}$ beschreibt ein minimales Spaltmaß zwischen dem Knetwerkzeug 7 und der Bottich-Innenwand 25 im betrachteten Achsabschnitt des Knetwerkzeugs 7.

Figur 2 zeigt Kräfte $F_{\varphi 1}$, $F_{\varphi 2}$, die der Teig in zwei Winkelpositionen $\varphi_{s1}$, $\varphi_{s2}$ des Knetwerkzeugs 7 im betrachteten Achsabschnitt auf das Knetwerkzeug 7 ausübt. Zu den beiden Winkelpositionen $\varphi_{s1}$, $\varphi_{s2}$ gehören zwei Spaltmaße $S_1$, $S_2$ zwischen dem Knetwerkzeug 7 im betrachteten Achsabschnitt und der Bottich-Innenwand 25. Die Kräfte, die auf das Knetwerkzeug 7 in diesen beiden Positionen $\varphi_{s1}$, $\varphi_{s2}$ auf das Knetwerkzeug 7 ausgeübt werden, haben neben den Tangentialkomponenten $F_{\varphi 1}$, $F_{\varphi 2}$, zusätzliche y-Komponenten $F_{y1}$, $F_{y2}$. Zudem ist in der Figur 3 noch schematisch das Drehmoment $M_\Omega$ eingezeichnet, wobei $\omega$ bzw. $\omega_S$ die Winkelgeschwindigkeit des Knetwerkzeugs 7 darstellt und somit ein Maß für die Drehfrequenz des Knetwerkzeugs 7 ist.

Die die Grundschwingung (Winkelgeschwindigkeit $\omega$) und die erste Oberwelle (Winkelgeschwindigkeit $2\omega$) des Drehmoments $M_\omega$ beschreibende Differentialgleichung beinhaltet als Koeffizienten eine Teig-Steifigkeit bzw. Teig-Elastizität $c_d$ und eine Teig-Viskosität $d_d$, die über den gesamten Knetprozess ermittelbar sind. Eine Einheit der Steifigkeit $c_d$ ist N/m. Eine Einheit der Teig-Viskosität $d_d$ ist Ns/m.

Die Differentialgleichung hat folgende Form ($\varphi = \omega t$, $d\varphi/dt = \omega$):

$$M(t) = c_d\, R_S\, (s_{min} + R_S)\, \cos(\omega t) - (c_d\, R_S^2/2)\, \sin(2\,\omega t) - (d_d\, R_S^2/2)\, \omega\, (\cos(2\,\omega t) + 1)$$

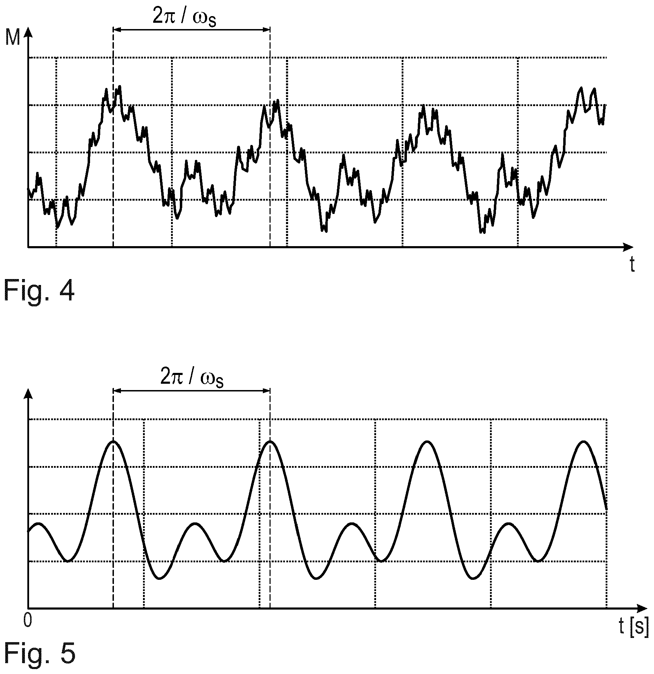

Figur 4 zeigt schematisch ein Messergebnis der zeitlichen Abhängigkeit des Drehmoments M, das vom Teig auf das Knetwerkzeug 7 ausgeübt wird und das mit der Drehmoment-Messeinheit 20 gemessen wird.

Figur 5 zeigt einen modellhaften zeitlichen Verlauf des Drehmoments M, das mithilfe entsprechender Fitparameter für die Teig-Steifigkeit $c_d$ und die Teig-Viskosität $d_d$ an die Messkurve nach Figur 4 angepasst wurde. Es ergibt sich ein periodischer Verlauf mit einer Periodendauer $T = 2t/\omega_s$, die der Zeitdauer entspricht, die ein voller Umlauf des Knetwerkzeugs 7 benötigt. Diese Zeitdauer T kann im Bereich zwischen 0,1 s und 1 s und beispielsweise im Bereich zwischen 0,15 und 0,45 s und insbesondere im Bereich 0,3 s liegen.

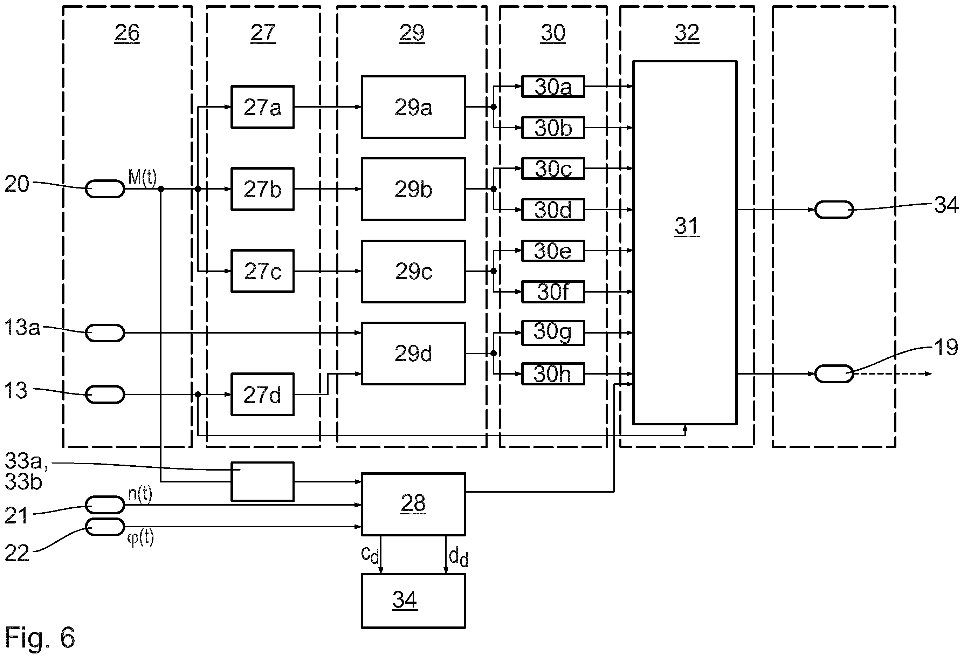

Mithilfe dieser Messdaten wird, wie anhand des Ablaufschemas nach Fig. 6 erläutert wird, mit der Analyseeinheit 23 ein zu erwartender Knetzeitraum bis zum Erreichen eines maximalen Drehmoments M, welches bei einem weiteren Betrieb der Teigknetvorrichtung 1 auf das Knetwerkzeug 7 wirkt, bestimmt. Weiterhin werden aus diesen zeitaufgelöst gemessenen Messdaten ein Teig-Elastizitäts- bzw. Steifigkeitsparameter und ein Teig-Viskositätsparameter als Teig-Ist-Parameter bestimmt.

Anhand der Figur 6 wird nachfolgend ein diese Bestimmung des zu erwartenden Knetzeitraums nutzendes Verfahren sowie ein die Bestimmung des Teig-Elastizitätsparameters und des Teig-Viskositätsparameters nutzendes Verfahren zum Betreiben der Teigknetvorrichtung 1 beschrieben.

Beim Knetbetrieb wird in einer Messphase 26 mithilfe der Drehmoment-Messeinheit 20 ein momentanes Drehmoment M bestimmt, welches auf das Knetwerkzeug 7 der Teigknetvorrichtung 1 wirkt. Weiterhin wird mithilfe der Drehzahl-Messeinheit 21 eine momentane Drehzahl bzw. eine Winkelgeschwindigkeit $\omega$ des Knetwerkzeugs 7 und mithilfe der Drehpositions-Messeinheit 22 eine momentane Drehposition $\varphi$ des Knetwerkzeugs 7 bestimmt.

In der Messphase 26 des Knetverfahrens wird weiterhin die Raumtemperatur mit dem Temperatursensor 13a und die Teigtemperatur mit dem Temperatursensor 13 bestimmt.

Die zeitaufgelösten Messwerte des Drehmoments und der Teigtemperatur werden dann in einer Vorfilterungsphase 27 mithilfe von Vorfilteralgorithmen 27a, 27b, 27c und 27d vorgefiltert. Das Motormomentsignal M(t) wird dabei parallel über die drei verschiedenen Vorfilteralgorithmen 27a, 27b, 27c vorgefiltert. Das noch ungefilterte Messergebnis des Motormoments M(t) wird wiederum parallel einem zu den sonstigen Bestimmungsalgorithmen parallelen erweiterten Kalman-Filteralgorithmus 28 übergeben, der seinerseits einen Bestimmungsalgorithmus darstellt. Mit dem erweiterten Kalman-Filteralgorithmus 28 lassen sich zudem ein Teig-Elastizitätsparameter und ein Teig-Viskositätsparameter als Teig-Ist-Parameter aus den zeitaufgelösten Messwerten bestimmen, wie nachfolgend noch erläutert wird.

Bei dem Filteralgorithmus 28 handelt es sich um ein erweitertes Kalman-Filter. Eine theoretische Beschreibung eines solchen Filters findet sich in: Dan Simon, Optimal State Estimation, Kalman, $H_\infty$ and Nonlinear Approaches, 2006.

In einer Extraktionsphase 29 wird ein Ausgangssignal des Vorfilteralgorithmus 27a an einen ersten Extraktionsalgorithmus 29a übergeben, der vom Typ der gleitenden Mittelwertbildung gestaltet ist.

Die Daten, die sich beim weiteren Vorfilteralgorithmus 27b ergeben, werden in der Extraktionsphase an einen weiteren Extraktionsalgorithmus 29b übergeben, bei dem es sich um einen Luenberger-Filteralgorithmus handelt und der als Störgrößenbeobachter ausgeführt ist. Ein derartiger Luenberger-Beobachter ist beschrieben in: D. G. Luenberger: Observing the State of a Linear System, IEEE Transaction on Military Electronics, Band 8, S. 74-80, 1964.

Das Filterergebnis des weiteren Vorfilteralgorithmus 27c wird in der Extraktionsphase 29 an einen weiteren Extraktionsalgorithmus 29c übergeben, bei dem es sich um einen Kalman-Filteralgorithmus handelt. Eine theoretische Beschreibung eines Kalman-Filters findet sich in: R. E. Kalman: A New Approach to Linear Filtering and Prediction Problems, Journal of Basic Engineering, 35-45, 1960.

Das ungefilterte Ergebnis der Raumtemperatur-Messung mithilfe des Temperatursensors 13a wie das über den Vorfilteralgorithmus 27d gefilterte Messergebnis der Teigtemperatur über den Temperaturfühler 13 werden in der Extraktionsphase an einen weiteren Extraktionsalgorithmus 29d übergeben, bei dem es sich ebenfalls um einen Luenberger-Filteralgorithmus handelt.

Die Ergebnisse der parallel arbeitenden Extraktionsalgorithmen 29a bis 29d werden nachfolgend jeweils gesplittet und in einer Nachfilterungsphase 30 in Nachfilterungs-Algorithmen 30a, 30b, 30c, 30e, 30f, 30g, 30h nachgefiltert. Anschließend werden diese nachgefilterten Ergebnisse der Nachfilterungs-Algorithmen 30a bis 30h an einen Überwachungsalgorithmus 31 übergeben. In den Überwachungsalgorithmus 31 kann als Input auch noch die vom Temperaturfühler 13 gemeldete Teigtemperatur ungefiltert eingehen.

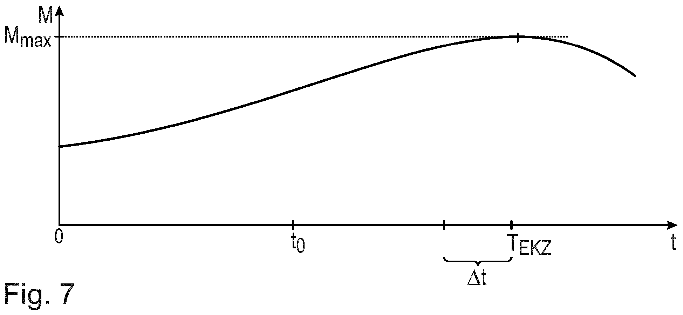

Die verschiedenen Bestimmungsalgorithmen 29a bis 29d sowie 28 führen zu voneinander unabhängigen Ergebnissen für einen zu erwartenden Knetzeitraum $T_{EKZ}$ (vgl. Fig. 7).

In einer Überwachungsphase 32 überwacht der Überwachungsalgorithmus 31 die Kriterien des Knetbetriebs, insbesondere, inwieweit der bestimmte, zu erwartende Knetzeitraum erreicht ist.

In dem parallel zu den vorstehend beschriebenen Bestimmungsalgorithmen arbeitenden Filteralgorithmus 28 gehen als Inputsignale neben dem gemessenen Drehmoment M(t) noch die Drehzahl n(t) und die Drehposition $\varphi$(t) ein, die von den Messeinheiten 20 bis 22 bereitgestellt werden.

Das ungefilterte Drehmomentsignal durchläuft parallel vor Eingang in den Filteralgorithmus 28 noch zwei Bandpass-Filter 33a, 33b. Eine Zentralfrequenz des Bandpass-Filters 33a kann im Bereich zwischen 3 und 4 Hertz (Grundschwingung $\omega$) liegen. Eine Zentralfrequenz des weiteren Bandpass-Filters 33b kann im Bereich zwischen 7 und 8 Hertz (erste Oberwelle $2\omega$) liegen. Das zunächst gesplittet beide Bandpass-Filter 33a, 33b durchlaufende Messsignal wird anschließend in Filteralgorithmus 28 wieder zusammengeführt. Zusätzlich zu den beiden Bandpass-Filtern 33a, 33b kann insbesondere in Zusammenhang mit der Mischphase noch ein weiterer Bandpass-Filter zum Einsatz kommen, der in der Zeichnung nicht dargestellt ist und eine Zentralfrequenz im Bereich zwischen 1 und 2 Hertz (Grundschwingung $\omega$ in der Mischphase) hat.

Im Filteralgorithmus 28 wird dann zunächst aus den gefilterten Drehmoment-Messsignalen bei den beiden Harmonischen $\omega$ und $2\omega$ ein zweidimensionaler Messvektor gebildet.

Aus den Komponenten des Messvektors sowie der ebenfalls gemessenen Drehzahl n(t) und der Drehposition $\varphi$(t) als weitere Komponenten wird einZustandsvektor mit insgesamt sieben Komponenten bestimmt. Im Rahmen der Verarbeitung über den erweiterten Kalman-Filter erfolgt eine Diskretisierung in entsprechend den sieben Zuständen des Zustandsvektors verkoppelte Systemfunktionen. Der Filteralgorithmus 28 mit dem erweiterten Kalman-Filter liefert also im Rahmen einer Schwingungsanalyse zusätzlich zu den anderen Algorithmen die Teig-Ist-Parameter Elastizität bzw. Steifigkeit $c_d$ und Viskosität $d_d$.

Die ersten drei Komponenten des Zustandsvektors entsprechen den drei Termen der obigen Differentialgleichung. Weitere zwei Komponenten des Zustandsvektors entsprechen der Teigsteifigkeit $c_d$ sowie der Teigviskosität $d_d$. Weitere zwei Komponenten des Zustandsvektors stehen für eine Phase der ersten harmonischen Schwingung $\omega$ zur rotierenden Position $\varphi$ des Knetwerkzeugs 7 und eine Phase zwischen jeweils den Summanten der zweiten Harmonischen $2\omega$ zu der berechneten Position $\varphi$ des Knetwerkzeugs 7 bei zweifacher Geschwindigkeit. Diese gesamte Zustandsfunktion mit insgesamt sieben Zustandsfunktionen wird dann dem Filteralgorithmus unterzogen.

Diese über den Filteralgorithmus 28 bestimmten Teig-Ist-Parameter ($c_d$, $d_d$) werden über ein Anzeigeelement 34 ausgegeben.

Diese Teig-Ist-Parameter können dann mit vorgegebenen Teig-Soll-Parametern $c_d{}^S$, $d_d{}^S$ zur Teig-Elastizität sowie zur Teig-Viskosität verglichen werden und der Knetbetrieb anhand des Ergebnisses dieses Vergleichs gesteuert werden. In den Vergleich der Teig-Ist-Parameter mit den vorgegebenen Teig-Soll-Parametern kann das Ermitteln eines Ist-Wertes einer Merit-Funktion eingehen. Die Merit-Funktion ist eine Funktion der Teig-Ist-Parameter. Der Ist-Wert der Merit-Funktion kann dann mit einem vorgegebenen Soll-Wert der Merit-Funktion verglichen werden.

Das vom Filteralgorithmus 28 gefilterte Outputsignal wird dann wiederum an den Überwachungsalgorithmus 31 übergeben. Der Überwachungsalgorithmus 31 gibt zum einen an das Anzeigeelement 34 ein Signal aus "zu erwartender Knetzeitraum erreicht" und andererseits ein Steuersignal an den Knetantriebsmotor 19 zum Beenden des Antriebs des Knetwerkzeugs 7, sobald eine vorbestimmte Knet-Betriebsdauer erreicht ist.

Figur 7 zeigt den typischen zeitlichen Verlauf des auf das Knetwerkzeug 7 wirkenden Drehmoments M bis zum Erreichen eines maximalen Drehmoments $M_{max}$.

Ziel des Betriebsverfahrens ist es, aus einem frühen zeitlichen Abschnitt, beispielsweise bis zu einem Zeitpunkt $t_0$, Messwerte, die während des Betriebs der Teigknetvorrichtung 1 sensorisch erfasst werden, so zu interpretieren, dass der zu erwartende Knetzeitraum $T_{EKZ}$ bis zum Erreichen eines maximalen Drehmoments $M_{max}$ bestimmt werden kann. Idealerweise liegt der Zeitpunkt $t_0$, ab dem der zu erwartende Knetzeitraum $T_{EKZ}$ bestimmt werden kann, sehr deutlich vor Ablauf dieses zu erwartenden Knetzeitraums, sodass beispielsweise bei $t_0 = T_{EKZ}/2$ mit dem Betriebsverfahren der zu erwartende Knetzeitraum $T_{EKZ}$ mit einer Fehlertoleranz bestimmt werden kann, die geringer ist als 10 Prozent, die geringer ist als 5 Prozent und die insbesondere geringer ist als 1 Prozent. Hierzu werden die regelmäßig während des Knetbetriebs stark variierenden Messwerte mittels bestimmter Algorithmen aufbereitet und insbesondere gefiltert und/oder geglättet.

Der Knetbetrieb kann entweder exakt nach Ablauf des bestimmten Knetzeitraums $T_{EKZ}$ oder alternativ eine definierte Zeitspanne Δt vor oder nach Ablauf des bestimmten Knetzeitraums $T_{EKZ}$ beendet werden. Hierbei muss natürlich gelten, dass der Beendigungszeitpunkt nach dem Bestimmungszeitpunkt $t_0$ liegt.

Bei der vorbestimmten Knetbetriebsdauer kann es sich exakt um den mithilfe des vorstehend erläuterten Betriebsverfahrens bestimmten Knetzeitraum handeln, also um den Knetzeitraum bis zum Erreichen des maximalen Drehmoments M.

Alternativ kann durch Ansteuerung des Knetantriebsmotors 19 über den Überwachungsalgorithmus 31 der Knetbetrieb eine definierte Zeitspanne vor Ablauf des mit dem Betriebsverfahren bestimmten Knetzeitraums beendet werden.

Kandidaten für den zu erwartenden Knetzeitraum ergeben sich als parallele Ergebnisse der nachgefilterten Extraktionsalgorithmen 29a bis 29d, die jeder für sich einen solchen zu den erwartenden Knetzeitraum-Kandidaten nach erfolgter Nachfilterung ausgeben. Aufgrund der gesplitteten Nachfilterung ergeben sich sogar für jeden dieser Extraktionsalgorithmen 29a bis 29d jeweils zwei zu erwartende Knetzeitraum-Kandidaten als Ergebnisse der Nachfilterungs-Algorithmen 30a bis 30h. Ein zusätzlicher Kandidat für den zu erwartenden Knetzeitraum ergibt sich als Ergebnis des Filteralgorithmus 28.

Der Filteralgorithmus 28 gibt zudem noch als Teigeigenschafts-Parameter aus einerseits die Teig-Steifigkeit $c_d$ und andererseits die Teig-Viskosität $d_d$. Diese Teigparameter können ebenfalls der Anzeigeeinheit 34 zugeführt werden.

Bei der Bestimmung des zu erwartenden Knetzeitraums kann derjenige Zeitraum-Kandidat als Resultat akzeptiert werden, der, innerhalb vorgegebener Toleranzgrenzen, von einer Mehrzahl der parallelen Bestimmungsalgorithmen 29a bis 29d, 28 ermittelt wurde. Eine derartige Mehrheits-Bestimmung wird auch als demokratische Bestimmung bezeichnet.

Auch die Teigfüllstandsmessung über den Füllstandssensor 24 kann in die Bestimmung des zu erwartenden Knetzeitraums eingehen.

**Patentansprüche**

1. Verfahren zum Betreiben einer Teigknetvorrichtung (1) mit folgenden Schritten:

- zeitaufgelöstes Messen eines momentanen Drehmoments, welches auf ein Knetwerkzeug (7) der Teigknetvorrichtung (1) wirkt,
- zeitaufgelöstes Messen einer momentanen Drehzahl des Knetwerkzeugs (7),
- zeitaufgelöstes Messen einer momentanen Drehposition des Knetwerkzeugs (7),
- Bestimmen eines Teig-Elastizitätsparameters ($c_d$) und eines Teig-Viskositätsparameters ($d_d$) als Teig-Ist-Parameter aus den Messwerten,
- Ausgeben von Teig-Zustandsdaten anhand der Messdaten und der bestimmten Teig-Ist-Parameter ($c_d$, $d_d$),
- wobei der Teig-Elastizitätsparameter ($c_d$) und der Teig-Viskositätsparameter ($d_d$) über eine Schwingungsanalyse der zeitaufgelösten Messdaten bestimmt werden,
- wobei in die Schwingungsanalyse eine Differentialgleichung der folgenden Form:

$$M(t) = c_d \, R_S \, (s_{min} + R_S) \cos(\omega t) - (c_d \, R_S^2/2) \sin(2 \, \omega t) - (d_d \, R_S^2/2) \, \omega$$

$$(\cos(2 \, \omega t) + 1)$$

mit

-- M: Drehmoment;
-- $R_s$: Radius des Knetwerkzeugs (7) im betrachteten Axialabschnitt;
-- $s_{min}$: minimales Spaltmaß zwischen dem Knetwerkzeug und einer Bottich-Innenwand im betrachteten Achsabschnitt des Knetwerkzeugs (7);
-- $\omega$: Winkelgeschwindigkeit des Knetwerkzeugs (7) eingeht, die eine Grundschwingung (Winkelgeschwindigkeit $\omega$) und eine erste Oberwelle (Winkelgeschwindigkeit $2\omega$) des momentanen Drehmoments beschreibt,

- Steuern der Teigknetvorrichtung (1) anhand der ausgegebenen Teig-Zustandsdaten.

2. Verfahren nach Anspruch 1, **gekennzeichnet durch** folgende Schritte:

- Vergleichen der Teig-Ist-Parameter ($c_d$, $d_d$) mit vorgegebenen Teig-Soll-Parametern ($c_d{}^S$, $d_d{}^S$),
- Steuern des Knetbetriebes anhand des Ergebnisses des Vergleichs.

3. Verfahren nach Anspruch 2, **dadurch gekennzeichnet, dass** beim Vergleichen der Teig-Ist-Parameter ($c_d$, $d_d$) mit den Teig-Soll-Parametern ($c_d{}^S$, $d_d{}^S$) folgende Schritte durchgeführt werden:

- Ermitteln eines Ist-Wertes einer Merit-Funktion, in die die Teig-Ist-Parameter ($c_d$, $d_d$) eingehen,
- Vergleichen des Ist-Wertes der Merit-Funktion mit einem vorgegebenen Soll-Wert der Merit-Funktion.

4. Verfahren zum Betreiben einer Teigknetvorrichtung (1) mit folgenden Schritten:

- zeitaufgelöstes Messen eines momentanen Drehmoments, welches auf ein Knetwerkzeug (7) der Teigknetvorrichtung (1) wirkt,
- zeitaufgelöstes Messen einer momentanen Drehzahl des Knetwerkzeugs (7),
- zeitaufgelöstes Messen einer momentanen Drehposition des Knetwerkzeugs (7),
- Bestimmen eines zu erwartenden Knetzeitraumes ($T_{EKZ}$) bis zum Erreichen eines maximalen Drehmoments ($M_{max}$), welches bei einem weiteren Betrieb der Teigknetvorrichtung (1) auf das Knetwerkzeug (7) wirkt, anhand des gemessenen Drehmoments, der gemessenen Drehzahl und der gemessenen Drehposition,
- Beenden eines Knetbetriebes für eine aktuell geknetete Teigcharge abhängig vom bestimmten Knetzeitraum ($T_{EKZ}$),

  --wobei das momentane Drehmoment über mindestens einen Vorfilter-Algorithmus (27a bis 27c) vorgefiltert und ein Ausgangssignal des Vorfilter-Algorithmus (27a bis 27c) an einen Extraktionsalgorithmus (29a bis 29c) übergeben wird, wobei als Extraktionsalgorithmus (29a bis 29c) zum Einsatz kommt:-eine gleitende Mittelwertbildung (29a) oder
  -- ein Luenberger-Filteralgorithmus (29b) oder
  -- ein Kalman-Filteralgorithmus (29c),

- wobei das Ergebnis des Extraktionsalgorithmus (29a bis 29c) anschließend in einem Nachfilterungs-Algorithmus nachgefiltert und ein Erreichen des maximalen Drehmoments zur Bestimmung des zu erwartenden Knetzeitraums überwacht wird.

5. Verfahren nach Anspruch 4, **dadurch gekennzeichnet, dass** der Knetbetrieb exakt nach Ablauf des bestimmten Knetzeitraums ($T_{EKZ}$) beendet wird.

6. Verfahren nach Anspruch 4, **dadurch gekennzeichnet, dass** der Betrieb eine definierte Zeitspanne ($\Delta t$) vor Ablauf des bestimmten Knetzeitraums ($T_{EKZ}$) beendet wird.

7. Verfahren nach einem der Ansprüche 4 bis 6, **dadurch gekennzeichnet, dass** in die Bestimmung des zu erwartenden Knetzeitraums ($T_{EKZ}$) mindestens zwei parallele Bestimmungsalgorithmen (29a bis 29d, 28) eingehen.

8. Verfahren nach Anspruch 7, **dadurch gekennzeichnet, dass** bei der Bestimmung des zu erwartenden Knetzeitraums ($T_{EKZ}$) derjenige Zeitraum als Resultat akzeptiert ist, der von einer Mehrzahl der parallelen Bestimmungsalgorithmen (29a bis 29d, 28) ermittelt wurde.

9. Verfahren nach einem der Ansprüche 1 bis 8, **dadurch gekennzeichnet, dass** ein Bestimmungsalgorithmus (29c, 28) zur Bestimmung der Teig-Ist-Parameter und/oder des zu erwartenden Knetzeitraumes ($T_{EKZ}$) einen Kalman-Filter einsetzt.

10. Verfahren nach einem der Ansprüche 1 bis 9, **dadurch gekennzeichnet, dass** ein Bestimmungsalgorithmus (29a) zur Bestimmung der Teig-Ist-Parameter und/oder des zu erwartenden Knetzeitraumes ($T_{EKZ}$) eine gleitende Mittelwertbildung einsetzt.

11. Verfahren nach einem der Ansprüche 1 bis 10, **dadurch gekennzeichnet, dass** in die Bestimmung der Teig-Ist-Parameter und/oder des zu erwartenden Knetzeitraumes ($T_{EKZ}$) eine Temperaturmessung eingeht.

12. Teigknetvorrichtung (1),

   - mit einem Teigbehälter (5),
   - mit einem im Teigbehälter (5) rotierenden Knetwerkzeug (7),
   - mit einem Knetwerkzeugantrieb (19),
   - mit einer Drehmoment-Messeinheit (20) zum zeitaufgelösten Messen eines momentanen Drehmoments, das auf das Knetwerkzeug (7) wirkt,
   - mit einer Drehzahl-Messeinheit (21) zum zeitaufgelösten Messen einer momentanen Drehzahl, mit der das Knetwerkzeug (7) rotiert,
   - mit einer Drehpositions-Messeinheit (22) zum zeitaufgelösten Messen einer momentanen Drehposition des Knetwerkzeugs (7),
   - mit einer Analyseeinheit (23), die mit der Drehmoment-Messeinheit (20), der Drehzahl-Messeinheit (21) und der Drehpositions-Messeinheit (22) in Signalverbindung steht, zum Auswerten von Drehmoment-Messergebnissen der Messeinheit (20),
   - wobei die Analyseeinheit (23) mit dem Knetwerkzeugantrieb (19) zum Steuern des Knetwerkzeugs (19) bis zum Erreichen einer vorbestimmten Knet-Betriebsdauer anhand der ausgewerteten Drehmoment-Messergebnisse in Signalverbindung steht,
   - wobei die Teigknetvorrichtung so ausgeführt ist, dass als Parameter zur Steuerung des Knetwerkzeugantriebs (19) zum Einsatz kommen:

      -- ein Teig-Elastizitätsparameter ($c_d$) und ein Teig-ViskositätsParameter ($d_d$) oder
      -- ein zu erwartender Knetzeitraum ($T_{EKZ}$) bis zum Erreichen eines maximalen Drehmoments ($M_{max}$), welches bei einem weiteren Betrieb der Knetvorrichtung (1) auf das Knetwerkzeug (7) wirkt,

   - wobei die Teigknetvorrichtung weiterhin so ausgeführt ist, dass der Teig-Elastizitätsparameter ($c_d$) und der Teig-Viskositätsparameter ($d_d$) über eine Schwingungsanalyse der zeitaufgelösten Messdaten bestimmt werden,
   - wobei in die Schwingungsanalyse eine Differentialgleichung der folgenden Form:

$$M(t) = c_d\, R_S\, (s_{min} + R_S)\cos(\omega t) - (c_d\, R_S^2/2)\sin(2\,\omega t) - (d_d\, R_S^2/2)\,\omega$$

$$(\cos(2\,\omega t) + 1)$$

   mit

      -- M: Drehmoment;
      -- $R_s$: Radius des Knetwerkzeugs (7) im betrachteten Axialabschnitt;
      -- $s_{min}$: minimales Spaltmaß zwischen dem Knetwerkzeug und einer Bottich-Innenwand im betrachteten Achsabschnitt des Knetwerkzeugs (7);
      -- $\omega$: Winkelgeschwindigkeit des Knetwerkzeugs (7) eingeht, die eine Grundschwingung (Winkelgeschwindigkeit $\omega$) und eine erste Oberwelle (Winkelgeschwindigkeit $2\omega$) des momentanen Drehmoments beschreibt,

- wobei die Analyseeinheit (23) so ausgeführt ist, dass der Knetwerkzeugantrieb (19) des Knetwerkzeugs (7) und damit die Teigknetvorrichtung (1) anhand von Messdaten der Messeinheiten (20 bis 22) und der Parameter zur Steuerung des Knetwerkzeugantriebs gesteuert wird.

**13.** Teigknetvorrichtung nach Anspruch 12, **gekennzeichnet durch** mindestens einen Temperatursensor (13, 13a).

**14.** Teigknetvorrichtung nach Anspruch 12 oder 13, **gekennzeichnet durch** einen Füllstandssensor (24) zur Bestimmung eines Teig-Füllstandes im Teigbehälter (5).

**Claims**

**1.** Method for operating a dough-kneading device (1) comprising the following steps:

- time-resolved measurement of a momentary torque acting on a kneading tool (7) of the dough-kneading device (1),
- time-resolved measurement of a momentary speed of the kneading tool (7),
- time-resolved measurement of a momentary rotational position of the kneading tool (7),
- determining a dough elasticity parameter ($c_d$) and a dough viscosity parameter ($d_d$) as the actual dough parameters from the measurement values,
- outputting dough status data based on the measurement data and the determined actual dough parameters ($c_d$, $d_d$),
- wherein the dough elasticity parameter ($c_d$) and the dough viscosity parameter ($d_d$) are determined by means of a vibration analysis,
- wherein in the vibration analysis a differential equation of the following form is included:

$$M(t) = c_d\, R_S\, (s_{min} + R_S)\, \cos(\omega t) - (c_d\, R_S{}^2/2)\, \sin(2\,\omega t) - (d_d\, R_S{}^2/2)\, \omega\, (\cos(2\,\omega t) + 1)$$

with

-- M being the torque;
-- $R_S$ being the radius of the kneading tool (7) in the considered axis section;
-- $s_{min}$ being a minimum gap dimension between the kneading tool (7) and as tub inner wall in the considered axis section of the kneading tool (7);
-- $\omega$ being the angular velocity of the kneading tool (7), which describes a fundamental oscillation (angular velocity $\omega$) and first upper wave (angular velocity $2\omega$) of the momentary torque,

- controlling the dough-kneading device (1) based on the output dough status data.

**2.** Method according to Claim 1, **characterized by** the following steps:

- comparing the actual dough parameters ($c_d$, $d_d$) with specified target dough parameters ($c_d{}^S$, $d_d{}^S$),
- controlling the kneading operation based on the result of the comparison.

**3.** Method according to Claim 2, **characterized in that** when comparing the actual dough parameters ($c_d$, $d_d$) with the target dough parameters ($c_d{}^S$, $d_d{}^S$), the following steps are carried out:

- determining an actual value of a merit function, with which the actual dough parameters ($c_d$, $d_d$) are associated,
- comparing the actual values of the merit function with a specified target value of the merit function.

**4.** Method for operating a dough-kneading device (1) comprising the following steps:

- time-resolved measurement of a momentary torque acting on a kneading tool (7) of the dough-kneading device (1),
- time-resolved measurement of a momentary speed of the kneading tool (7),

- time-resolved measurement of a momentary rotational position of the kneading tool (7),
- determining an expected kneading period ($T_{EKZ}$) until reaching a maximum torque ($M_{max}$) which, in the event of further operation of the dough-kneading device (1), acts on the kneading tool (7), based on the measured torque, the measured speed and the measured rotational position,
- ending a kneading operation for a currently kneaded batch of dough depending on the determined kneading period ($T_{EKZ}$),

-- wherein the momentary torque is pre-filtered via at least one pre-filter algorithm (27a bis 27c) and an output signal of the pre-filter algorithm (27a bis 27c) is transferred to an extraction algorithm (29a bis 29c), wherein as an extraction algorithm (29a bis 29c):
-- a floating mean value formation (29a) or
-- a Luenberger-filter algorithm (29b) or
-- a Kalman-filter algorithm (29c) is used,

- wherein the result of the extraction algorithm (29a to 29c) is subsequently post-filtered in a post-filtering-algorithm and it is monitored when the maximum torque has been reached to determine the expected kneading period.

5. Method according to Claim 4, **characterized in that** the kneading operation is ended exactly after the expiry of the determined kneading period ($T_{EKZ}$).

6. Method according to Claim 4, **characterized in that** the operation is ended following a defined period of time ($\Delta t$) before the expiry of the determined kneading period ($T_{EKZ}$).

7. Method according to one of Claims 4 to 6, **characterized in that** at least two parallel determination algorithms (29a to 29d, 28) are included in the determination of the expected kneading period ($T_{EKZ}$).

8. Method according to Claim 7, **characterized in that**, in determining the expected kneading period (Tnnz), as a result, that period which was determined by a plurality of the parallel determination algorithms (29a to 29d, 28) is accepted.

9. Method according to one of Claims 1 to 8, **characterized in that** a determination algorithm (29c, 28) for determining the actual dough parameters and/or the expected kneading period ($T_{EKZ}$) uses a Kalman filter.

10. Method according to one of Claims 1 to 9, **characterized in that** a determination algorithm (29a) for determining the actual dough parameters and/or the expected kneading period ($T_{EKZ}$) uses a floating mean value formation.

11. Method according to one of Claims 1 to 10, **characterized in that** a temperature measurement is included in the determination of the actual dough parameters and/or the expected kneading period ($T_{EKZ}$).

12. Dough-kneading device (1),

- with a dough container (5),
- with a kneading tool (7) rotating within the dough container (5),
- with a kneading tool drive (19),
- with a torque measuring unit (20) for time-resolved measurement of a momentary torque acting on the kneading tool (7),
- with a speed measuring unit (21) for time-resolved measurement of a momentary speed at which the kneading tool (7) rotates,
- with a rotational position measuring unit (22) for time-resolved measurement of a momentary rotational position of the kneading tool (7),
- with an analysis unit (23) connected to the torque measuring unit (20), the speed measuring unit (21) and the rotational position measuring unit (22) on a signal level for the analysis of torque measurement results of the measuring unit (20),
- wherein the analysis unit (23) is connected to the kneading tool drive (19) on a signal level to control the kneading tool (19) until an expected kneading period has been achieved on the basis of the evaluated torque measurement results,
- wherein the dough-kneading device (1) is configured such that the following parameters are applied to control the kneading tool drive (19):

-- a dough elasticity parameter ($c_d$) and a dough viscosity parameter ($d_d$) or
-- an expected kneading period ($T_{EKZ}$) until the maximum torque ($M_{max}$) has been reached which acts onto the kneading tool (7) in the event of a further operation of the dough-kneading device (1),

- wherein the dough-kneading device (1) is further configured such that the dough elasticity parameter ($c_d$) and the dough viscosity parameter ($d_d$) are determined via a vibration analysis of the time-resolved measurement data,
- wherein in the vibration analysis a differential equation of the following form is included:

$$M(t) = c_d \, R_S \, (s_{min} + R_S) \cos(\omega t) - (c_d \, R_S^2/2) \sin(2 \, \omega t) - (d_d \, R_S^2/2) \, \omega$$

$$(\cos(2 \, \omega t) + 1)$$

with

-- M being the torque;
-- $R_S$ being the radius of the kneading tool (7) in the considered axis section;
-- $s_{min}$ being a minimum gap dimension between the kneading tool (7) and as tub inner wall in the considered axis section of the kneading tool (7);
-- $\omega$ being the angular velocity of the kneading tool (7), which describes a fundamental oscillation (angular velocity $\omega$) and first upper wave (angular velocity $2\omega$) of the momentary torque,

- wherein the analysis unit (23) is configured such that the kneading tool drive (19) of the kneading tool (7) and thus the dough-kneading device (1) is controlled on the basis of measurement data of the measuring units (20 to 22) and the parameters for controlling the kneading tool drive (19).

13. Dough-kneading device according to Claim 12, **characterized by** at least one temperature sensor (13, 13a).

14. Dough-kneading device according to Claim 12 or 13, **characterized by** a level sensor (24) for determining a dough level in the dough container (5).

## Revendications

1. Procédé de fonctionnement d'un dispositif de pétrissage de pâte (1) comprenant les étapes suivantes :

- mesure résolue en temps d'un couple instantané qui agit sur un outil de pétrissage (7) du dispositif de pétrissage de pâte (1),
- mesure résolue en temps d'une vitesse de rotation momentanée de l'outil de pétrissage (7),
- mesure résolue en temps d'une position de rotation momentanée de l'outil de pétrissage (7),
- détermination d'un paramètre d'élasticité de pâte ($c_d$) et d'un paramètre de viscosité de pâte ($d_d$) en tant que paramètres de pâte réels à partir des valeurs mesurées,
- sortie de données d'état de pâte à partir des données de mesure et des paramètres réels de pâte ($c_d$, $d_d$) déterminés,
- dans lequel le paramètre d'élasticité de pâte ($c_d$) et le paramètre de viscosité de pâte ($d_d$) sont déterminés par une analyse vibratoire des données de mesure résolues en temps,
- dans lequel on introduit dans l'analyse vibratoire une équation différentielle de la forme suivante :

$$M(t) = c_d \, R_S \, (s_{min} + R_S) \cos(\omega t) - (c_d \, R_S^2/2) \sin(2 \, \omega t) - (d_d \, R_S^2/2) \, \omega$$

$$(\cos(2 \, \omega t) + 1)$$

où

-- M représent la couple ;

-- $R_s$ représent le rayon de l'outil de pétrissage (7) dans la section axiale considérée ;

-- $s_{min}$ représent l'espace minimal entre l'outil de pétrissage et une paroi intérieure de la cuve dans la section axiale considérée de l'outil de pétrissage (7) ;

-- $\omega$ représent la Vitesse angulaire de l'outil de pétrissage (7), qui décrit une oscillation fondamentale (vitesse angulaire $\omega$) et une première harmonique (vitesse angulaire $2\omega$) du couple instantané,

- commande du dispositif de pétrissage de pâte (1) à l'aide des données d'état de pâte sorties.

2. Procédé selon la revendication 1, **caractérisé par** les étapes suivantes :

- comparaison des paramètres réels de pâte ($c_d$, $d_d$) avec les paramètres de consigne de pâte ($c_d{}^S$, $d_d{}^S$) prédéfinis,
- contrôle du pétrissage à l'aide du résultat de la comparaison.

3. Procédé selon la revendication 2, **caractérisé en ce que** lors de la comparaison des paramètres réels de pâte ($c_d$, $d_d$) avec les paramètres de consigne de pâte ($c_d{}^S$, $d_d{}^S$), les étapes suivantes sont effectuées :

- détermination d'une valeur réelle d'une fonction de mérite dans laquelle entrent les paramètres de pâte réels ($c_d$, $d_d$),
- comparaison de la valeur réelle de la fonction de mérite avec une valeur de consigne prédéfinie de la fonction de mérite.

4. Procédé de fonctionnement d'un dispositif de pétrissage de pâte (1) comprenant les étapes suivantes :

- mesure résolue en temps d'un couple instantané qui agit sur un outil de pétrissage (7) du dispositif de pétrissage de pâte (1),
- mesure résolue en temps d'une vitesse de rotation momentanée de l'outil de pétrissage (7),
- mesure résolue en temps d'une position de rotation momentanée de l'outil de pétrissage (7),
- détermination d'une période de pétrissage ($T_{EKZ}$) attendue jusqu'à ce qu'un couple maximal ($M_{max}$), qui agit sur l'outil de pétrissage (7) lors d'un fonctionnement ultérieur du dispositif de pétrissage de pâte (1), soit atteint, à l'aide du couple mesuré, de la vitesse de rotation mesurée et de la position de rotation mesurée,
- terminaison d'un pétrissage pour une charge de pâte actuellement pétrie en fonction de la période de pétrissage ($T_{EKZ}$) déterminée,

-- dans lequel le couple instantané est préfiltré par au moins un algorithme de préfiltrage (27a à 27c) et un signal de sortie de l'algorithme de préfiltrage (27a à 27c) est transmis à un algorithme d'extraction (29a à 29c), dans lequel on utilise comme algorithme d'extraction (29a à 29c) :
-- un calcul de moyenne glissante (29a), ou
-- un algorithme de filtrage de Luenberger (29b), ou
-- un algorithme de filtrage de Kalman (29c),

- dans lequel le résultat de l'algorithme d'extraction (29a à 29c) est ensuite post-filtré dans un algorithme de post-filtrage et une atteinte du couple maximal est surveillée pour déterminer le temps de pétrissage attendu.

5. Procédé selon la revendication 4, **caractérisé en ce que** le pétrissage s'arrête exactement à la fin de la période de pétrissage ($T_{EKZ}$) déterminée.

6. Procédé selon la revendication 4, **caractérisé en ce que** le fonctionnement est arrêté pendant une période définie ($\Delta t$) avant la fin de la période de pétrissage ($T_{EKZ}$) déterminée.

7. Procédé selon l'une quelconque des revendications 4 à 6, **caractérisé en ce qu'**au moins deux algorithmes de détermination parallèles (29a à 29d, 28) entrent dans la détermination de la période de pétrissage ($T_{EKZ}$) attendue.

8. Procédé selon la revendication 7, **caractérisé en ce que** lors de la détermination de la période de pétrissage ($T_{EKZ}$) attendue, la période qui a été déterminée par une pluralité des algorithmes de détermination parallèles (29a à 29d, 28) est acceptée comme résultat.

9. Procédé selon l'une quelconque des revendications 1 à 8, **caractérisé en ce qu'**un algorithme de détermination (29c, 28) utilise un filtre de Kalman pour déterminer les paramètres de pâte réels et/ou la période de pétrissage ($T_{EKZ}$) attendue.

10. Procédé selon l'une quelconque des revendications 1 à 9, **caractérisé en ce qu'**un algorithme de détermination (29a) utilise un calcul de moyenne glissante pour déterminer les paramètres de pâte réels et/ou la période de pétrissage ($T_{EKZ}$) attendue.

11. Procédé selon l'une quelconque des revendications 1 à 10, **caractérisé en ce que** la détermination des paramètres réels de pâte et/ou de la période de pétrissage ($T_{EKZ}$) attendue inclut une mesure de la température.

12. Dispositif de pétrissage de pâte (1),

- avec un récipient à pâte (5),
- avec un outil de pétrissage (7) tournant dans le récipient à pâte (5),
- avec un entraînement de l'outil de pétrissage (19),
- avec une unité de mesure de couple (20) pour la mesure résolue en temps d'un couple instantané qui agit sur l'outil de pétrissage (7),
- avec une unité de mesure de vitesse de rotation (21) pour la mesure résolue en temps d'une vitesse de rotation momentanée à laquelle l'outil de pétrissage (7) tourne,
- avec une unité de mesure de position de rotation (22) pour la mesure résolue en temps d'une position de rotation momentanée de l'outil de pétrissage (7),
- avec une unité d'analyse (23), qui est en liaison de signal avec l'unité de mesure de couple (20), l'unité de mesure de vitesse de rotation (21) et l'unité de mesure de position de rotation (22), pour l'évaluation des résultats de mesure de couple de l'unité de mesure (20),
- dans lequel l'unité d'analyse (23) est en liaison de signal avec l'entraînement de l'outil de pétrissage (19) pour commander l'outil de pétrissage (19) jusqu'à ce qu'une durée de fonctionnement de pétrissage prédéterminée soit atteinte à l'aide des résultats de mesure de couple évalués,
- dans lequel le dispositif de pétrissage de pâte est réalisé de telle sorte que les paramètres suivants sont utilisés pour commander l'entraînement de l'outil de pétrissage (19) :

-- un paramètre d'élasticité de pâte ($c_d$) et d'un paramètre de viscosité de pâte ($d_d$) ou
-- une période de pétrissage ($T_{EKZ}$) attendue jusqu'à ce qu'un couple maximal ($M_{max}$) soit atteint, lequel agit sur l'outil de pétrissage (7) lors d'un fonctionnement ultérieur du dispositif de pétrissage (1),

- dans lequel le dispositif de pétrissage de pâte est en outre conçu de telle sorte que le paramètre d'élasticité de pâte ($c_d$) et le paramètre de viscosité de pâte ($d_d$) sont déterminés par l'intermédiaire d'une analyse vibratoire des données de mesure résolues en temps,
- dans lequel on introduit dans l'analyse vibratoire une équation différentielle de la forme suivante :

$$M(t) = c_d\, R_S\, (s_{min} + R_S)\, \cos(\omega t) - (c_d\, R_S^2/2)\, \sin(2\,\omega t) - (d_d\, R_S^2/2)\, \omega$$

$$(\cos(2\,\omega t) + 1)$$

où

-- M représent la couple ;
-- $R_s$ représent le rayon de l'outil de pétrissage (7) dans la section axiale considérée ;
-- $s_{min}$ représent l'espace minimal entre l'outil de pétrissage et une paroi intérieure de la cuve dans la section axiale considérée de l'outil de pétrissage (7) ;
-- $\omega$ représent la Vitesse angulaire de l'outil de pétrissage (7), qui décrit une oscillation fondamentale (vitesse angulaire $\omega$) et une première harmonique (vitesse angulaire $2\omega$) du couple instantané,

- dans lequel l'unité d'analyse (23) est conçue de telle sorte que l'entraînement de l'outil de pétrissage (19) de l'outil de pétrissage (7) et ainsi le dispositif de pétrissage de pâte (1) sont commandés à l'aide de données de mesure des unités de mesure (20 à 22) et des paramètres pour la commande de l'entraînement de l'outil de pétrissage.

**13.** Dispositif de pétrissage de pâte selon la revendication 12, **caractérisé par** au moins un capteur de température (13, 13a).

**14.** Dispositif de pétrissage de pâte selon la revendication 12 ou 13, **caractérisé par** un capteur de niveau de remplissage (24) pour déterminer un niveau de remplissage de pâte dans le récipient à pâte (5).

Fig. 1

EP 3 613 290 B1

Fig. 3

Fig. 2

$2\pi / \omega_S$

M

t

Fig. 4

$2\pi / \omega_S$

0

t [s]

Fig. 5

Fig. 6

EP 3 613 290 B1

19

Fig. 7

EP 3 613 290 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102018214278
- DE 102010042542 A1
- JP 2008145132 A
- US 4747690 A
- US 5472273 A
- US 5906432 A
- EP 0428241 A1


**In der Beschreibung aufgeführte Nicht-Patentliteratur**

- **KESSLER, JAN HENNING ; TRÄCHTLER, ANSGAR.** Control of Pareto Points for Self-Optimizing Systems with Limited Objective Values. *The 19th IFAC World Congress, Cape Town, South Africa,* 20140824
- **D. G. LUENBERGER.** Observing the State of a Linear System. *IEEE Transaction on Military Electronics,* 1964, vol. 8, 74-80
- **R. E. KALMAN.** A New Approach to Linear Filtering and Prediction Problems. *Journal of Basic Engineering,* 1960, 35-45